(19) Europäisches Patentamt
European Patent Office
Office européen des brevets

(11) Publication number: **0 197 784**
**A1**

(12) # EUROPEAN PATENT APPLICATION

(21) Application number: 86302533.4

(22) Date of filing: 04.04.86

(51) Int. Cl.[4]: C 12 N 11/14
C 12 P 13/20, C 12 N 9/50
C 12 P 13/22
//G01N33/50, C12R1:07

(30) Priority: 04.04.85 US 720015

(43) Date of publication of application:
15.10.86 Bulletin 86/42

(84) Designated Contracting States:
AT BE CH DE FR GB IT LI LU NL SE

(71) Applicant: GENEX CORPORATION
16020, Industrial Drive
Gaithersburg Maryland 20877(US)

(72) Inventor: Swann, Wayne E.
5392, Storm Drift
Columbia Maryland 21045(US)

(72) Inventor: Nolf, Carol A.
14347, Rosetree Court
Silver Spring Maryland 20906(US)

(74) Representative: Holmes, Michael John et al,
Frank B. Dehn & Co. European Patent Attorneys Imperial House 15-19 Kingsway
London, WC2B 6UZ,(GB)

(54) **Method for immobilizing a biological material.**

(57) The present invention provides a method for immobilizing a biological material by preparing an insolubilized biological material composite comprising the steps of:

(a) coating glass fibers of matrices with a mixture of the biological material and a polymer; and

(b) insolubilizing the polymer by applying a cross-linking agent and/or a condensing agent to the coated glass fibers or matrices.

EP 0 197 784 A1

# METHOD FOR IMMOBILIZING A BIOLOGICAL MATERIAL

This invention relates to a method for immobilizing a biological material. More particularly, the invention relates to the immobilization of a biological material wherein the biological material is mixed with a polymer, coated onto glass fibers or matrices and insolubilized by adding a cross-linking and/or condensing agent.

Biological materials, such as enzymes or enzyme-producing microorganisms or cells, are often used as catalysts for synthetic reactions and for analytical techniques. Such catalysts are desirable because they operate with high specificity and efficiency under generally mild reaction conditions.

Because enzymes and other biocatalysts are generally water-soluble, they are suited for use in batch-type reaction systems. Reuse of such enzymes and other biocatalysts is limited because of difficulties in recovering those materials from the spent reaction medium in active or usable form. Moreover, the materials tend to remain in the prepared product as contaminants. In order to avoid these problems, methods have been developed to immobilize biological materials which exhibit catalytic activity on insoluble solid supports. Immobilization is intended to result in a stabilized biological material which can withstand the rigors of repeated or continuous use.

Several immobilization systems for biological materials have been reported. Enzymes have been immobilized by absorption onto insoluble materials such as charcoal, glass, cellulose, calcium phosphate gel, montmorillonite and organic ion-exchange resins among others. Immobilization also has been achieved by entrapment within starch and acrylamide gels, covalent attachment between enzymes and insoluble organic polymers, and covalent attachment between enzyme molecules themselves.

The processes of the prior art often result in products of reduced biological activity when compared with those of the corresponding unbound biological material. These biological materials are known to be sensitive to thermal and chemical denaturation or inactivation. The loss of biological activity often results when immobilizing operations are carried out under harsh conditions which can be particularly problematic when polymer condensation reactions are involved. Furthermore, the products resulting from prior art methods are often disadvantageous with respect to their hydrophilicity, strength, durability, and porosity.

Other problems can occur in immobilization systems which employ compressible materials. When a column of immobilized biological material is compressed, channeling, which causes uneven flow during use, may occur. There also may be partial degradation of the column or pressure buildup in the system due to the compressibility of the support material.

A further problem with insoluble solid support systems known in the art is their reliance on porous materials with high overall surface area to trap a large amount of biological material. These porous materials may be capable of containing more biological material

than other systems, but some substrate molecules are larger than the pores of the support material. In these cases, the substrate cannot diffuse easily into the porous material to react with the biological material.

Porous supports are also problematic when smaller substrate molecules are employed because even molecules small enough to diffuse into the pores may not readily do so in large enough quantities to react sufficiently with the immobilized biological material.

It is therefore an object of the present invention to develop a method of immobilizing biological materials that does not reduce the biological activity of the products.

It is a further object of this invention to develop a method of immobilizing biological materials wherein the insoluble solid support is incompressible.

It is a further object of this invention to develop a method of immobilizing biological materials wherein there is a high amount of accessible surface area such that a substrate can readily react with the biological materials.

It is a further object of this invention to develop a method of immobilizing biological materials wherein the resulting products exhibit excellent strength, durability, and biological stability.

Yet another object of the invention is to provide a method of immobilizing a high quantity of biological material per unit volume of final support.

According to one aspect of the present invention, we provide a method of immobilizing a biological material by preparing an insolubilized biological material composite comprising the steps of: (a) coating glass fibers or matrices with a mixture of the biological material and a polymer; and (b) insolubilizing the polymer by

applying a cross-linking agent and/or a condensing agent to the coated glass fibers or matrices.

It now has been discovered that biological materials can be immobilized on an incompressible support which eliminates the problems of the prior art while maintaining a high degree of catalytic activity in the biological material.

The method of the present invention comprises immobilizing biological materials by preparing an insolubilized biological material composite by coating glass fibers or matrices with a mixture of the biological material and a polymer, for example in an aqueous medium, and insolubilizing the polymer by adding a cross linking agent and/or a condensing agent. Coating as defined in this invention means any method of applying the biological material to the glass fibers or matrices. The biological material may be in a dry powder form or in an aqueous medium to be mixed with the polymer. Very little loss of activity occurs when the composite is prepared, and such composites exhibit excellent strength and durability. Furthermore, when the polymer is cross-linked or condensed, the hydrophilicity of these materials can be adjusted by varying the extent of crosslinking or condensation. Large numbers of tiny glass fibers or filaments, each of which provides surface area on which biological material can be coated, can provide a large total external surface area and avoid the problems encountered with highly porous material wherein substrate molecules are unable to reach and react with the biological material in the pores due to interparticle diffusion. Glass fibers also are incompressible so that channeling, partial degradation of the insoluble support and pressure build up is inhibited. The method of the

present invention produces a composite which can be separated from reaction mixtures by simple filtration or used in continuous reaction processes such as those wherein a reacting substrate flows through a packed column reactor.

In accordance with the method of the present invention, different types of glass fibers or filaments may be used as the support, including single fibers, matrices of fibers, woven-mats, cloth, strand fibers which are continuous or chopped individual fibers, or wool. These glass fibers have good mechanical characteristics, are not compressible, and have no thermal, chemical or biological degrading effect on biological materials. The glass fibers or filaments have a high surface area because every gram of glass provides up to 1 square meter of surface area. The external surface area depends on fiber diameter. When smaller diameters of fiber are used, there is greater utilizable surface area because more fibers can be placed in the same amount of space. The fiber diameter can be as small as 2 microns and may be between 2-10 microns. Generally, the diameter will be between 8 and 20 microns. Using the method of this invention, there is more uniform crosslinking than with other support materials.

The glass fibers are coated with a mixture of the biological material to be insolubilized and a polymer, then treated with a cross-linking agent or a condensing agent to insolubilize the polymer and link the biological material to the polymer. The polymer optionally may be combined with a carboxylic acid to form a water soluble pre-polymer before it is applied to the glass fibers or filaments. The coatings on the glass fibers or filaments should be thin coatings, comprising less than 50% of the total weight of the glass fiber core.

The method of the present invention allows the preparation of a wide variety of biological material composites. The biological material can include enzymes, microbial cells, antigens, antibodies, antibiotics, coenzymes, plant cells, animal cells, bacteria, yeasts, fungi, tissue cultures or mixtures thereof. Whole cells can be entrapped within a matrix of glass fibers by allowing the cells to be absorbed into the matrix after coating the fibers and then applying a cross-linking agent. The cross linking of the polymer holds the cells within the matrix.

It has been discovered that glass fibers or matrices make excellent carriers for the immobilization of biological materials. These fibers or matrices can retain biomass much like porous materials by entrapping the biomass within a matrix of woven fibers without the attendant disadvantages of porous supports. The glass fibers or matrices exhibit good flow characteristics and provide a high surface area to which the biological materials can be applied, thus ensuring that the biological material will be readily available and accessible to react as required. The immobilization support resulting from this method of immobilizing biological material is rigid and highly active.

The polymers used in the method and composites of the present invention generally vary in molecular weight, depending on reaction conditions. A variety of polymeric materials can be used in the method of this invention, including polyalkyleneimines, polysaccharides, polyacrylamide, polyurethane, alginate and carageenan. Preferred polymers are polyalkyleneimines, especially polyethyleneimines, polypropyleneimines, polybutylene-imines, and polypentyleneimine, with polyethyleneimine being especially preferred.

Polyalkyleneimines can be synthesized by the acid-catalyzed addition polymerization of alkyleneimine monomers. Polyethyleneimine (PEI) is a preferred polyalkyleneimine because it currently is readily available at relatively low cost, and it functions well in the condensation reactions employed in the present method. Polyethyleneimine is produced by ring-opening polymerization of ethyleneimine in the presence of catalysts, such as mineral acids. The polymer is highly branched and contains primary, secondary and tertiary amino groups. PEI is water-soluble and is conveniently employed as an aqueous solution e.g. containing 50% by weight of PEI; upon crosslinking or condensation of the polymer chains, a water-insoluble product results.

The polyethyleneimine can be crosslinked with an amine cross-linking agent to impart additional stability and strength. This treatment results in entrapped biological material, with some crosslinking between the polyalkyleneimine and free amine groups of the biological material. Suitable cross-linking agents include glutaraldehyde, diisocyanates, polyisocyanates, 2,4,6-trichloro-s-triazine, bisoxirane, bisimidate, divinylsulfone, 1,5-difluoro-2,4-dinitrobenzene and epoxides. Glutaraldehyde is preferred for this purpose.

The polymer chosen generally is added to the composite in an amount sufficient to substantially coat the glass fibers or filaments and this amount will vary depending on the nature of the biological materials and the physical properties desired. The amount of crosslinking agent employed is related to the amount of polymer.

When the polymer is polethyleneimine, a highly efficient method of condensation utilizes a polycarboxylic acid (PCA) to bridge amine groups on adjacent PEI chains. Condensing agents, preferably carbodiimides, readily effect the condensation. The

reactions involved in making the condensed polyethyleneimine of the present invention are illustrated below:

(1) 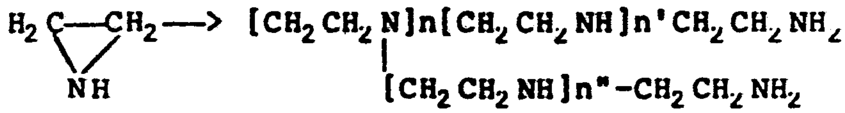

(2) 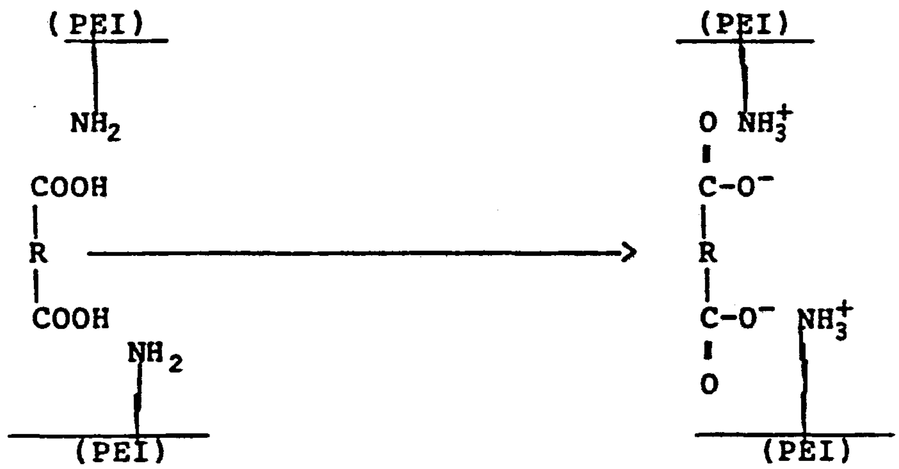

(3) 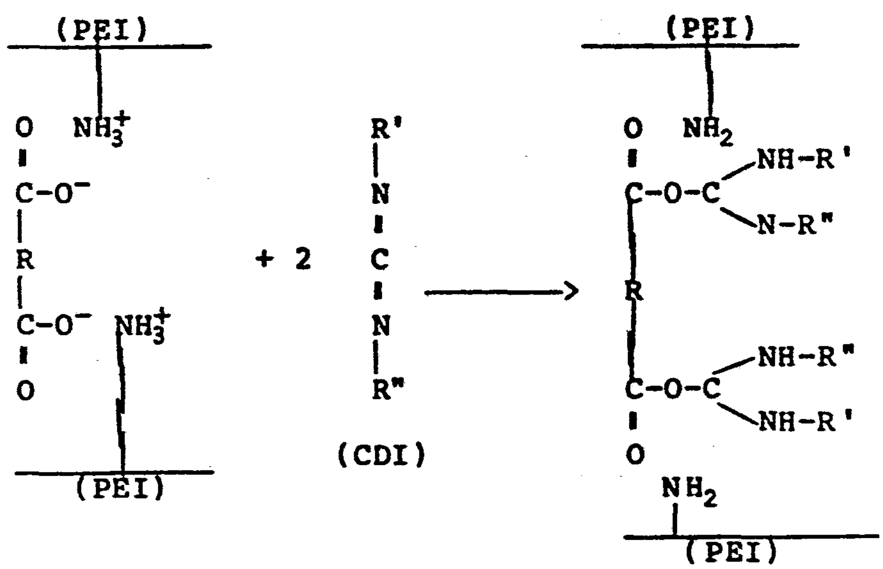

(4) 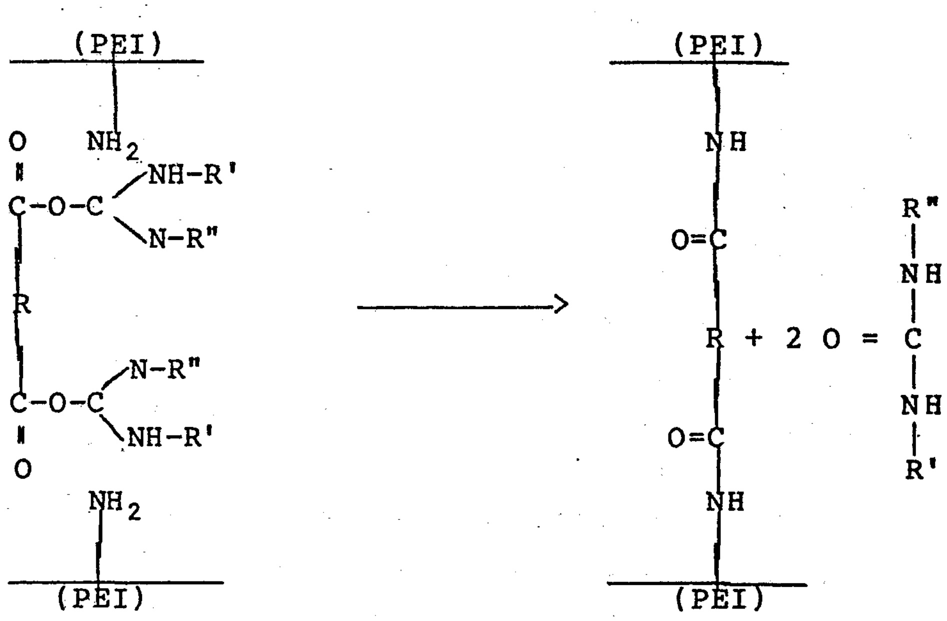

Reaction (1) illustrates the polymerization of ethyleneimine to form PEI having a branched-chain structure, wherein n and n' are integers greater than 0 and n" may be 0 (indicating that the [$CH_2CH_2NH$] group is absent) or greater than 0. Reaction (2) shows the formation of a salt of the amine groups of PEI with a polycarboxylic acid, wherein R can be a substituted or unsubstituted hydrocarbon group; reactions (3) and (4) show the condensation of the PEI and polycarboxylic acid, using a carbodiimide condensing agent. R and R' are hydrocarbon groups which, along with other reactants and conditions of the illustrated reactions, are described more fully below.

In general, polycarboxylic acids suited for use in the present invention may be substituted or unsubstituted carboxylic acids having at least two carboxylic groups. Examples of polycarboxylic acids that can be employed in the methods and composites of the present invention include adipic, azelaic, 1,11-undecanedioic, 1,12-dodecanedioic, traumtic, pentadecanedioic, hexadecanedioic, sebacic, suberic, glutaric, malonic,

pimellic, succinic, malic, maleic, glutamic, aspartic, oxalic, fumaric and polyaspartic acid. Dicarboxylic acids are preferred for use in the present invention and include maleic acid, succinic acid, glutaric and adipic acid. Higher polycarboxylic acids can be any substance that contains two or more carboxylic acid groups, and include high molecular weight polymeric materials, such as polyaspartic acid, having a molecular weight of from 5,000 to 50,000. The condensation reactions are generally exothermic, therefore, the reaction mixtures are advantageously cooled to a temperature that is not deleterious to the biological material being immobilized, e.g., about 37° or lower.

The molar ratio of polycarboxylic acid to polyalkyleneimine (PCA:PAI) can vary widely, because of the variation in molecular weight of the reactants. Generally, such ratios range from 1:20 to 1:0.0005.

As noted above, to effect condensation of polyalkyleneimine chains through polycarboxylic acids, a condensing agent is advantageously employed. Generally, any condensing agent that catalyzes or facilitates the reaction of amines and carboxylic acids can be used. Examples of such condensing agents include N-ethyl-5-phenyl-isoazolium-3-sulfonate, n-ethoxycarbonyl-2-ethoxy-1,2-dihydroquinoline, and various carbodiimides. Carbodiimide condensing agents are preferred, and those that can be used in the composition of the present invention have the formula R'-N=C=N-R" where R' and R" are hydrocarbyl groups containing from 3 to about 20 carbon atoms, preferably from about 5 to about 12 carbon atoms. Such condensing agents include 1-ethyl-3,3-dimethylaminopropyl carbodiimide, dicyclohexyl carbodiimide, 1-cyclohexyl-3-(2-morpholinoethyl) carbodiimidemetho-p-toluene sulphonate, or salts thereof. Carbodiimide condensing agents are added to the reaction

mixture in a condensing amount, which generally is substantially a stoichiometric amount; e.g. from about 0.2 to 3 times, preferably from about 0.5 to 1.5, a stoichiometric amount. Each carbodiimide molecule reacts with a single acid group of the polycarboxylic acid. For example, carbodiimide to dicarboxylic acid molar ratios of about 2:1 are generally used in the method of the present invention. Upon addition of the condensing agent at room temperature, noticeable polymerization results within thirty seconds, and is generally complete within about two hours.

When the polyethyleneimine has been insolubilized by the addition of a condensing agent, an optional post treatment step involves crosslinking the condensed, coated glass fibers or filaments with an amine cross-linking agent, such as glutaraldehyde, as described above, to provide additional strength and stability to the final composite. Depending upon the type of polymer chosen, a variety of condensing and cross-linking agents may be selected from those known in the art to strengthen the composite. Preferred combinations for this invention are PEI and glutaraldehyde, PEI and epoxy compounds, or PEI and a polycarboxylic acid in conjunction with a carbodiimide.

In the following examples, immobilization procedures of this invention are described in greater detail. These examples set forth various embodiments of the invention but are not to be construed as limiting.

## Example I

A crude preparation of glucoamylase enzyme was immobilized onto chopped glass fibers. The enzyme, 4.0 grams, was mixed with 0.45 grams of 50% aqueous polyethyleneimine until uniform, and then mixed with 2.4 grams of fibers. Glutaraldehyde (4cc of a 25% solution in water) was added

slowly and stirred until evenly distributed. After drying, the material was washed in water and packed into a column with a bed volume of 8.0cc. A 30% partially hydrolyzed starch substrate was passed through the column at 3.6cc/hr (0.45 SV/h). The effluent was monitored for glucoamylase activity by measuring the amount of glucose with a colorimetric determination using o-toluidine. The initial productivity of the column was 105 grams glucose/liter bed volume/hour. The column was operated continuously for 65 days at 60°C, and at the end of this time the productivity was 57 gm/liter bed volume/hr.

Example II

*E. coli* cell paste, having an aspartase activity of 46,200 units, was used in a whole cell immobilization on fiberglass. The support was prepared by twisting together several lengths of 408 filament/strand glass fibers to form matrices, each having a total weight of about 0.5 grams. A coating mixture was prepared consisting of 6 grams of the *E. coli* cell paste mixed with 24cc distilled water and 0.75 grams of 50% aqueous polyethyleneimine. When the mixture appeared uniform, 2.5cc was pipetted evenly onto each 0.5 gram section of vertically suspended fibers, and allowed to dry for 15 minutes. Glutaraldehyde then was pipetted over top of the coating (0.42cc of a 5% solution in water) and left to dry overnight. In all, a total of 2 grams of glass were used with 10cc of coating.

The material was cut into 0.5cm pieces and packed into a column with a final bed volume of 7.6cc and a density of 0.26 grams cell paste/cc. The column then was used to convert ammonium fumarate to L-aspartic acid. A 1.8M solution of ammonium fumarate with 1mM magnesium sulfate, pH 8.5, at 37°C was passed through the column at

4.2cc/hr (0.55 SV/h). The effluent was monitored for aspartase activity by measuring the disppearance of fumaric acid using a spectrophotometer at a wavelength of 240nM. An average of 99.2% conversion of the substrate was maintained for 5 days of continuous column operation.

## Example III

The *E. coli* paste of Example II also was used for whole cell immobilization onto a fiber matrix with a density of 0.102 gram/cc. The cell paste (20 grams) was mixed with 2.5 grams polyethyleneimine until uniform and then mixed with 0.7 grams of the fiberglass matrix. Glutaraldehyde (3.3 grams of a 25% solution) was then added slowly, with stirring, and the mixture was allowed to dry for 1 hour.

The material was packed into a column having a final bed volume of 22.4cc and a cell paste density of 0.5 grams/cc. The column converted 99.8% of a 1.8M ammonium fumarate substrate at 16.4cc/hr. single pass (0.74 SV/h) and 37°C.

## Example IV

Viable cells of a Bacillus species known to produce alkaline protease enzyme were immobilized onto a fiber matrix having a density of 0.115 grams/cc. Cell paste was harvested from a fermentation broth with alkaline protease activity of 16,000 delft units/ml. The cell paste (2 grams) was mixed aseptically with 3cc sterile water and 0.25 grams of 50% aqueous polyethyleneimine. Then 0.7 grams of the fiber matrix was added and allowed to soak for 15 minutes. The fiberglass was removed and placed in a beaker of 10 ml of a 5% glutaraldehyde solution to soak for 5 minutes, then washed in the substrate (a 10% diluted fermentation medium).

The immobilized whole cell material was placed in a batch reactor at 37°C containing 20 ml of media consisting of a carbon and nitrogen source and a continuous supply of dissolved oxygen. Samples were taken for 24 hours to monitor the production of alkaline protease by measuring enzymatic activity using a casein substrate. Alkaline protease production increased during the bioreactor operation demonstrating production of the enzyme by the immobilized cells.

## Example V

Rhodotorula rubra cells containing the enzyme phenylalanine ammonia-lyase were immobilized onto a fiberglass matrix material. The matrix material was composed of individual fibers in bundles and the overall density of the matrix was 0.115 gm/cc. A solution containing R. rubra cells, at 10% dry weight, was prepared, and polyethyleneimine (PEI) was added to the solution until a 10% PEI concentration resulted. Two grams of the glass fiber matrix material then was allowed to soak in the solution.

The material was removed and soaked in a 12.5% glutaraldehyde solution for 5 minutes, then removed and thoroughly washed. The R. rubra cells did not visually wash out of the support material during washings. The immobilized R. rubra cells then were used to make L-phenylalanine from ammonium cinnamate. Fifty milliliters of a 60mM cinnamate, 6M ammonia, pH 10.0 substrate solution was placed in a beaker with the immobilized R. rubra cells for 18 hours at room temperature. Over 2 grams of L-phenylalanine was produced per liter of initial substrate.

CLAIMS

1. A method for immobilizing a biological material by preparing an insolubilized biological material composite comprising the steps of:

(a) coating glass fibers of matrices with a mixture of the biological material and a polymer; and

(b) insolubilizing the polymer by applying a cross-linking agent and/or a condensing agent to the coated glass fibers or matrices.

2. A method as claimed in claim 1 wherein the mixture of the biological material and the polymer employed to coat the glass fibers or matrices is in an aqueous medium.

3. A method as claimed in claim 1 or claim 2 wherein the polymer is selected from the group consisting of polyalkyleneimines, polysaccharides, polyacrylamide, polyurethane, alginate and carageenan.

4. A method as claimed in claim 3 wherein the polymer is a polyalkyleneimine.

5. A method as claimed in claim 4 wherein the polyalkyleneimine polymer is selected from the group consisting of polyethyleneimine, polypropyleneimine, polybutyleneimine and polypentyleneimine.

6. A method as claimed in claim 5 wherein the polymer is polyethyleneimine.

7. A method as claimed in any one of claims 4 to 6 wherein a cross-linking amount of an amine cross-linking agent is applied to said coated glass fibers or matrices to immobilize the biological material.

8. A method as claimed in any one of claims 4 to 6 wherein said polvalkyleneimine polymer is mixed with a polycarboxylic acid to produce a water soluble pre-polymer before it is coated onto said glass fibers or matrices and the said water soluble pre-polymer after application to the glass fibers or matrices is condensed by adding a condensing amount of a carbodiimide under condensing conditions.

9. A method as claimed in claim 8 further comprising modifying the insolubilized biological material composite by post-treatment with an amine cross-linking agent to impart additional strength and stability to the composite.

10. A method as claimed in claim 8 or claim 9 wherein the polycarboxylic acid is selected from the group consisting of adipic acid, azelaic acid, 1,11-undecanedioic acid, 1,12-dodecanedioic acid, traumtic acid, pentadecanedioic acid, hexadecanedioic acid, sebacic acid, suberic acid, glutaric acid, malonic acid, pimellic acid, succinic acid, malic acid, maleic acid, glutamic acid, aspartic acid, oxalic acid, fumaric acid and polyaspartic acid.

11. A method as claimed in any one of claims 8 to 10 wherein the carbodiimide is selected from the group consisting of 1-ethyl-3,3-dimethylaminopropyl carbodiimide, dicyclohexyl carbodiimide and 1-cyclohexyl 3-(2-morpholinoethyl)carbodiimidemetho-p-toluene sulphonate and salts thereof.

12. A method as claimed in claim 7 wherein said amine cross-linking agent is selected from the group consisting of glutaraldehyde, diisocyanates, polyisocyanates, 2,4,6-trichloro-S-triazine, bisoxirane, bissimidate, divinyl-sulfone, 1,5-difluoro-2,4-dinitrobenzene and epoxides.

13. An insolubilized biological material composite comprising glass fibers or matrices having on the surface thereof a mixture of a biologically active material and a cross-linked polymer.

14. An insolubilized biological material composite as claimed in claim 13 prepared by a method as claimed in claim 7.

15. An insolubilized biological material composite as claimed in claim 13 prepared by a method as claimed in claim 8.

16. A composite as claimed in any one of claims 13 to 15 comprising glass fibers having a diameter between 2 and 20 microns.

17. A composite as claimed in any one of claims 13 to 16 wherein the biologically active material is selected from the group consisting of enzymes, microbial cells, antigens, antibodies, antibiotics, co-enzymes, bacteria, yeast, fungi, plant cells, animal cells and tissue cultures.

18. A composite as claimed in claim 17 wherein the biologically active material is glucoamylase.

19. A composite as claimed in claim 17 wherein the biologically active material is aspartase or aspartase-containing microbial cells.

20. A composite as claimed in claim 17 wherein the biologically active material is an alkaline protease-producing microorganism of the genus Bacillus.

21. A composite as claimed in claim 17 wherein the biologically active material is phenylalanine

ammonia lyase or phenylalanine ammonia lyase-containing microbial cells.

22. A method for making aspartic acid which comprises contacting, under aspartic acid-producing conditions, a substrate solution containing ammonium fumarate with an insolubilized biological material composite as claimed in claim 19.

23. A method for making an alkaline protease which comprises contacting, under alkaline protease-producing conditions, a suitable fermentation medium with an insolubilized biological material composite as claimed in claim 20.

24. A method for making L-phenylalanine which comprises contacting, under L-phenylalanine-producing conditions, a substrate solution containing ammonium cinnamate with an insolubilized biological material composite as claimed in claim 21.

European Patent Office

# EUROPEAN SEARCH REPORT

0197784
Application number

EP 86302533.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| Y | DE - A1 - 3 323 078 (GENEX CORP.)<br>* Claims 1-3,6,9,10,13,14,23,31, 32,34,36; page 19, lines 18-34 *<br>-- | 1,3-13, 17,19, 21,22, 24 | C 12 N 11/14<br>C 12 P 13/20<br>C 12 N 9/50<br>C 12 P 13/22<br>//G 01 N 33/50<br>C 12 R 1:07 |
| Y | US - A - 4 266 029 (BRANNER-JORGENSEN)<br>* Claims 1,2 *<br>-- | 1 | |
| A | US - A - 4 434 228 (SWANN)<br>* Claims 1-5,8-11,22,23,26,28, 32,33 *<br>---- | 1,3-6, 9-12, 17,19, 21,22, 24 | |
| | | | **TECHNICAL FIELDS SEARCHED (Int. Cl 4)** |
| | | | C 12 N<br>C 12 P<br>G 01 N<br>A 61 K |

The present search report has been drawn up for all claims

| Place of search | Date of completion of the search | Examiner |
|---|---|---|
| VIENNA | 18-06-1986 | BECKER |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document